# EUROPEAN PATENT APPLICATION

(11) **EP 2 657 810 A1**
(43) Date of publication of application: **30.10.2013**
(21) Application number: 11850662.5
(22) Date of filing: 24.12.2011
(51) Int. Cl.: G06F 3/023

(54) **METHOD AND DEVICE FOR CONSTRUCTING ORGANIC CHEMISTRY STRUCTURAL FORMULA**

(30) Priority: 24.12.2010 CN 201010622069
(71) Applicant: Peking University Founder Group Co., Ltd, Haidian District Beijing 100871 (CN); Beijing Founder Electronics Co., Ltd., Haidian District, Beijing 100085 (CN); Peking University Founder R&D Center, Beijing 100871 (CN)
(72) Inventor: ZHAO, Zhigang, Beijing 100085 (CN)
(74) Representative: Harrison Goddard Foote LLP
(86) International application number: PCT/CN2011/084595
(87) International publication number: WO 2012/083886

(57) **Abstract**

The application provides a method for constructing an organic chemical structure formal. The method may comprise a step of acquiring a character string inputted through a keyboard by a user, a step of searching an organic chemical structure mapped by the acquired character string in a preset code mapping set, and a step of constructing the organic chemical structure formula according to the searched out organic chemical structure. The application further provides an apparatus for constructing an organic chemical structure formal comprising: an acquiring module configured to acquire a character string inputted through a keyboard by a user; a searching module configured to search an organic chemical structure mapped with the acquired character string in a preset code mapping set; and a constructing module configured to construct the organic chemical structure formula according to the searched out organic chemical structure. According to the present application, operation efficiency of inputting organic chemical structure formals can be improved.

## Description

### Technical Field

The present application relates to a field of digital typesetting, in particular, to a method and an apparatus for constructing an organic chemical structure formula.

### Background

In the existing interactive chemical typesetting software, the organic chemical structure formals are usually typeset through a number of controls in a graph interface. A user is required to take various controls in the graph interface by operating the mouse to input the organic chemical structure formals. It seems to be intuitive, but it is actually difficult for the user to manage the method fast and it is more difficult to improve the operating speed.

### Summary

The present application intends to provide a method and an apparatus for constructing an organic chemical structure formula so as to at least solve complicated operations of inputting organic chemical structure formulas in the prior art.

According embodiments of the present application, a method for constructing an organic chemical structure formula is provided. The method comprises:
acquiring, through a keyboard, a character string inputted by a user;
searching an organic chemical structure mapped by the acquired character string in a preset code mapping set; and
constructing the organic chemical structure formula according to the searched out organic chemical structure.

According embodiments of the present application, an apparatus for constructing an organic chemical structure formal is provided. The apparatus comprises:
an acquiring module configured to acquire, through a keyboard, a character string inputted by a user;
a searching module configured to search an organic chemical structure mapped by the acquired character string in a preset code mapping set; and
a constructing module configured to construct the organic chemical structure formula according to the searched out organic chemical structure.

According to a method and an apparatus for constructing an organic chemical structure formal provided in embodiments of the present application, complicated operations of inputting organic chemical structure formals may be simplified and operation efficiency of inputting organic chemical structure formals may be improved by using a keyboard.

### Brief Description of the Drawing

The drawings described herein are used to provide a further understanding to the present application and constitute a part of this specification. Exemplary embodiments of the present application and their descriptions serve to explain the present application and do not constitute improper limitation on the present application. In the drawings:

Fig. 1 is a flowchart illustrating a method for constructing an organic chemical structure formula according to an embodiment of the present application.

Fig. 2 illustrates a screenshot of a blank chemical block according to a preferred embodiment of the present application.

Fig. 3 is a schematic diagram illustrating a definition of a data structure according to a preferred embodiment of the present application.

Fig. 4 illustrates a screenshot of the focus at an atom according to a preferred embodiment of the present application.

Fig. 5 illustrates a screenshot of the focus at a chemical bond according to a preferred embodiment of the present application.

Fig. 6 is a schematic diagram illustrating a connection mode of bonds according to a preferred embodiment of the present application.

Fig. 7 is a schematic diagram illustrating a connection mode 1 of atoms according to a preferred embodiment of the present application.

Fig. 8 is a schematic diagram illustrating a connection mode 2 of atoms according to a preferred embodiment of the present application.

Fig. 9 is a schematic diagram illustrating a connection mode 3 of atoms according to a preferred embodiment of the present application.

Fig. 10 is a schematic diagram illustrating an apparatus for constructing an organic chemical structure formula according to an embodiment of the present application

### Detailed Description

Hereinafter, the present application will be explained in detail with reference to the accompanying drawings in connection with embodiments thereof.

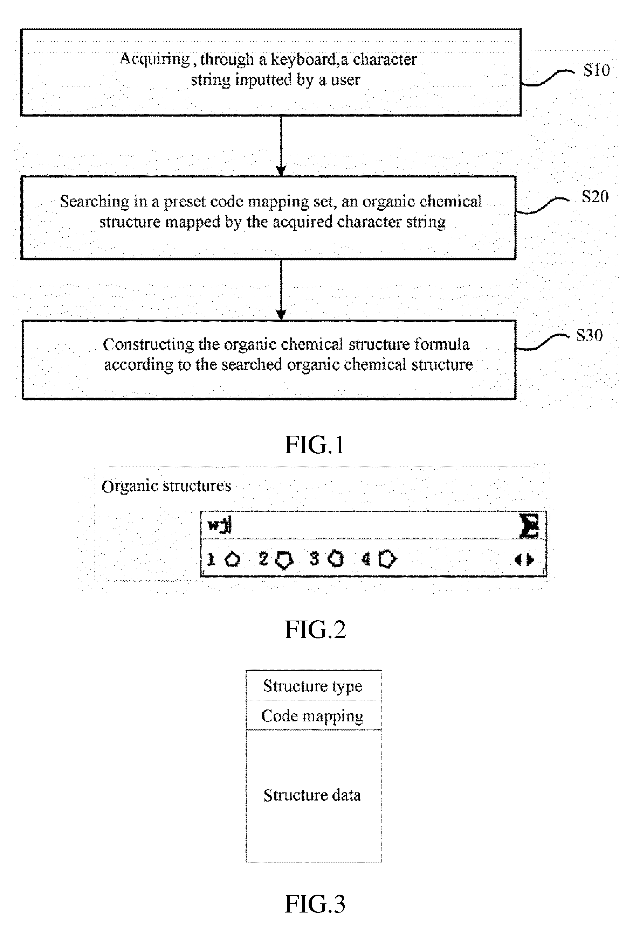

Fig. 1 is a flowchart illustrating a method for constructing an organic chemical structure formula according to an embodiment of the present application, the method may comprise:
a step S10 of acquiring, through a keyboard, a character string inputted by a user;
a step S20 of searching, in a preset code mapping set, an organic chemical structure mapped by the acquired character string; and
a step S30 of constructing the organic chemical structure formula according to the searched organic chemical structure.

In the prior art, various organic chemical structures may only be inputted by operations for a mouse. However, in the present embodiment, various organic chemical structures are generated by mapping the input from the keyboard so as to generate the chemical structure formulas, so that the efficiency for an interactive chemical typesetting software can be improved by operations of keyboard, without a mouse or buttons for finding and operating types of a large number of various organic chemical structures, to facilitate the user in obtaining various expected structures, so that usability and efficiency of the chemical typesetting can be improved.

Preferably, in the step S10, an interactive chemical typesetting interface is provided and an input window in the interface is activated, and then it monitors the inputs from the keyboard. Fig. 2 illustrates a screenshot of a blank chemical block (i.e., no-focus) according to a preferred embodiment of the present application. In Fig. 2, a user may input "wj" through an input window to output various pentagon ring-shaped structures to facilitate the user's selections. Therefore, organic chemical structures and connection modes thereof may be encoded and shown to the user through the inputting interface so that area of the interface can be saved and operations can be more direct and fast, and then usability and efficiency of the chemical typesetting may be improved. The input window of the keyboard may be activated passively, that is, it may be started by explicit operations of the user and be closed automatically.

Fig. 3 is a schematic diagram illustrating a definition of a data structure according to a preferred embodiment of the present application. Preferably, the method further includes a step of presetting the code mapping set that includes a plurality of metadata, wherein the metadata includes: 1) a structure type item for indicating that a focus is no-focus, an atom or a bond; 2) a code mapping item for indicating a character string mapping an organic chemical structure, for example, codes for benzene ring structure may be *bh, benhuan* and etc.; and 3) a structure data item for indicating an mapped organic chemical structure in which internal data, data of connection points or sides of the organic chemical structure may be stored. This data structure is simple and can be achieved easily by computer programming. In the actual application, the method according to the present application is initiated by the application program to transmit the current state, the organic chemical structure is searched according to the current focus and codes, and then data of the searched organic chemical structures are sent back to the program and corresponding analysis and connection are performed.

Preferably, in step S20, it is determined that the current focus in the interactive chemical typesetting interface is no-focus (i.e., a blank chemical block), an atom or a bond, and it looks up the structure type item and code mapping item of the metadata in the code mapping set according the determined focus and the inputted character string, and then it extracts the organic chemical structure from the searched structure data item of the metadata.

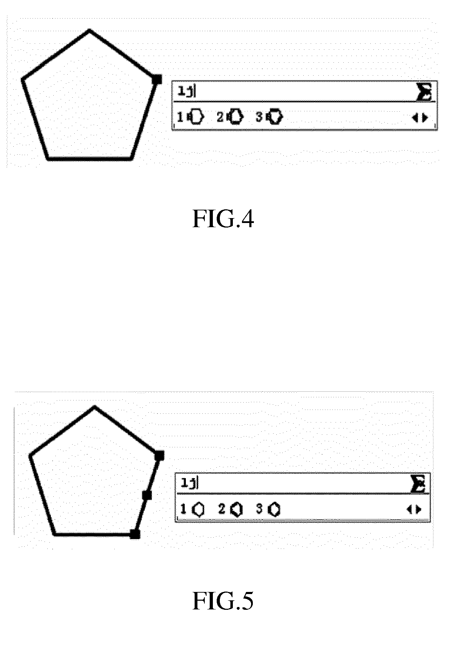

Fig.4 illustrates a screenshot of the focus at an atom according to a preferred embodiment of the present application. Fig. 5 illustrates a screenshot of the focus at a chemical bond according to a preferred embodiment of the present application. In the preferred embodiment, various organic chemical structure formulas can be edited by dividing a focus into three conditions including no-focus, an atom or a bond.

Preferably, step S30 may comprise a step of outputting an organic chemical structure in the structure data item from the interactive chemical typesetting interface, when it is determined that the focus is no-focus. In the case of a blank chemical block, it is difficult to select corresponding connection mode according to the conditions of a layout (i.e., the interactive chemical typesetting interface) and difficult to calculate the position of the chemical structure to be inserted into the layout according to the selected reference point. Therefore, it is not necessary to provide connection information under the state where the blank chemical block was defined. The pattern and the position to be inputted into the layout should be consistent with the displayed pattern of the input content and the position in the dialog box of the method, i.e. the input window. For example, if the content displayed in the dialog box is 1 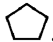 , the displayed pattern at the layout should be 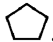 , rather than the pattern of 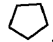 . The content shown in figure 2 is the defined content including various kinds of positions for various ring-typed structures.

Preferably, when it is determined that the focus is an atom, the step 30 further comprises the following three connection modes 1-3.

The connection mode 1 (i.e., a many-to-many connection mode): in this mode, a new chemical bond is constructed between the atom at the focus and an atom of the searched organic chemical structure, the bond length of the new chemical bond is determined as a default bond length, and then the angle position of the new chemical bond is determined according to the position of the atoms in the interactive chemical typesetting interface and information of the connected bonds.

The connection mode 2 (i.e., a one-to-one connection mode): In this mode, the atom at the focus and the chemical bond connected thereto are deleted and replaced with contents of the organic chemical structure.

The connection mode 3 (i.e., a many-to-one or one-to-many connection mode): in this step, the atom at the focus and an atom of the searched organic chemical structure are connected and combined directly, and the position and angle of the connected structures are determined according to the information in the interactive chemical typesetting interface.

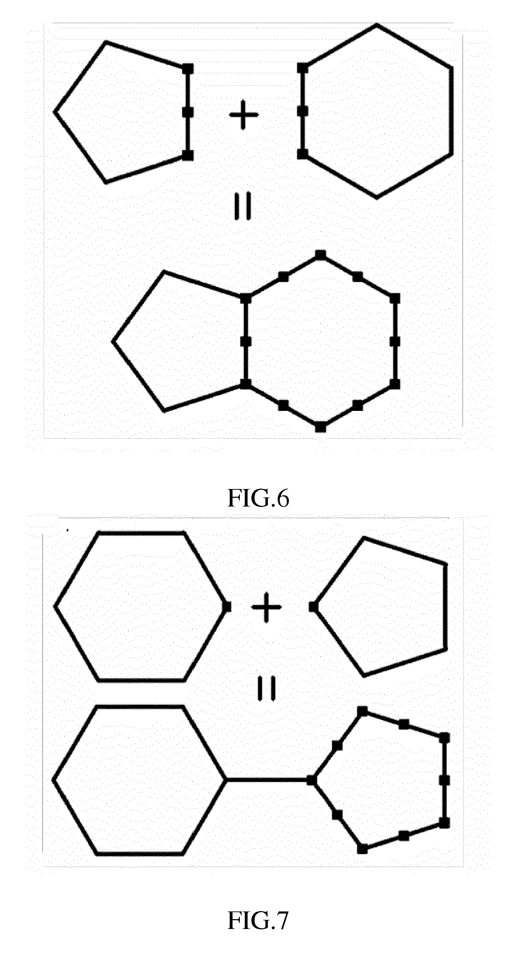

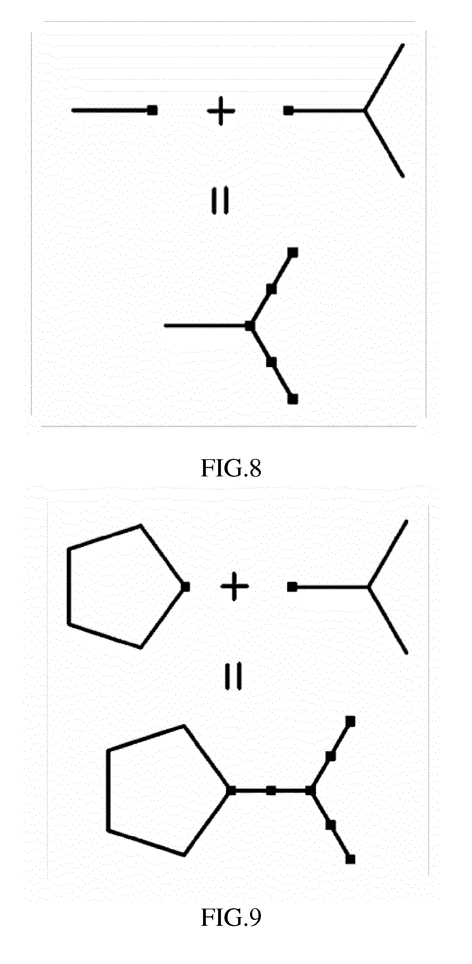

The focus is the single connecting atom point provided in the atom state for the connection between the selected points in the same layout. Since a chemical bond includes two atoms and it is difficult to identify from the selected atoms in the layout which one of the two atoms is to be connected actually, an atom and a bond cannot be connected. Various ring-shaped structures of connection points are provided in Fig.3. Figs. 7-9 are schematic diagrams respectively illustrating the connection modes 1-3 of atoms according to a preferred embodiment of the present application, in which the structure behind the plus sign is the searched organic chemical structure.

Preferably, in step S30,when it is determined that the focus is a bond, the bond and the searched organic chemical structure are connected to be a new bond, the bond length of the new bond is determined according to information of the bond at the focus, and then the position of the new bond is determined according to the corresponding angel information.

The focus is information of a bond for connection provided in the chemical bond state. Based on the similar reason as above, a bond and an atom cannot be connected, while two bonds can be connected. Various ring-shaped structures for one connection side are provided in Fig. 4. Fig. 6 is schematic diagram illustrating a connection mode of bonds according to a preferred embodiment of the present application. For the inputted contents, the bond length of the chemical bond of the connected structure may be calculated according to information of the selected chemical bond in the layout and the position of the connected bond may be calculated according to the corresponding angel information.

In the above-mentioned embodiments shown in Figs. 2-4, when a plurality of organic chemical structures are searched out, those organic chemical structures are shown in the input window and codes are provided in the window for selection. The contents accomplished by the method according to the present application are determined depending on the focus states of the current organic chemical structures on the layout. If the same code is inputted, the contents outputted when the focus is an atom may be different from those when the focus is a chemical bond. Then the user can easily input the expected organic chemical structure only through the button selection. Furthermore, the content is associated with the focus, so the rate of coincident code is low and the corresponding content can be found fast.

Preferably, the method further comprises the following steps: interpreting the character string as an operating command when the focus is a plurality of atoms and/or bonds; and executing the operating command for the plurality of atoms and/or bonds. For example, a character string "del" may be interpreted as a delete operation and the plurality of atoms and/or bonds may be deleted. For example, a character string "xz" may be interpreted as a rotate operation and the plurality of atoms and/or bonds may be rotated. According to the preferred embodiment, organic chemical structures can be edited easily.

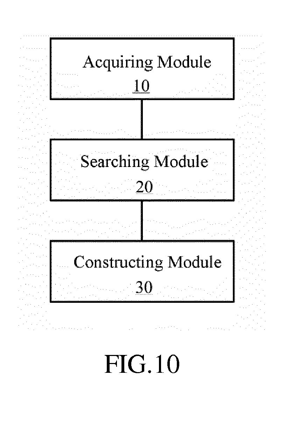

Fig. 10 is a schematic diagram illustrating an apparatus for constructing an organic chemical structure formal according to an embodiment of the present application. The apparatus comprises an acquiring module 10 configured to acquire a character string inputted by a user through a keyboard, a searching module 20 configured to search an organic chemical structure mapped by the acquired character string in a preset code mapping set, and a constructing module 30 configured to construct the organic chemical structure formula according to the searched organic chemical structure.

The usability and efficiency of chemical typesetting can be improved by the apparatus.

Preferably, the acquiring module 10 may comprise an interface module configured to provide an interactive chemical typesetting interface, a window module configured to activate an input window for the keyboard, and a monitor module configured to monitor inputs from the keyboard.

Preferably, the apparatus may further comprise an encoding module configured to preset the code mapping set including a plurality of metadata, wherein the metadata includes: 1) a structure type item for indicating a focus being no-focus, an atom or a bond; 2) a code mapping item for indicating a character string mapping an organic chemical structure; and 3) a structure data item for indicating an mapped organic chemical structure.

Preferably, the searching module 20 may comprise:
a focus module configured to determine whether the current focus in the interactive chemical typesetting interface is no-focus, an atom or a bond;
a lookup module configured to search the structure type item and code mapping item of the metadata in the code mapping set according the determined focus and the inputted character string; and
an extracting module configured to extract the organic chemical structure from the searched out structure type item of the metadata.

Preferably, the constructing module 30 may comprise the following modules:
a no-focus module configured to output an organic chemical structure in the structure data item through the interactive chemical typesetting interface, when it is determined that the focus is no-focus; and
an atom module configured, when it is determined that the focus is an atom, to construct chemical structure by the following connection modes:
   a many-to-many connection mode in which a new chemical bond is constructed between the atom at the focus and an atom of the searched out organic chemical structure, the bond length of the new chemical bond is determined as a default bond length, and then the angle position of the new chemical bond is determined according to the position of the atoms in the interactive chemical typesetting interface and information of the connected bonds;
   a one-to-one connection mode in which the atom at the focus and the chemical bond connected thereto are deleted and replaced with contents of the organic chemical structure; and
   a many-to-one or one-to-many connection mode in which the atom at the focus and an atom of the searched out organic chemical structure are connected and combined directly, and the position and angle of the connected atoms are determined according to information in the interactive chemical typesetting interface.

The constructing module 30 may further comprise:
a bond module configured, when it is determined that the focus is a bond, to connect the bond and the searched organic chemical structure to be a new bond, determine the bond length of the new bond according to information of the bond at the focus and then determine the position of the new bond according to the corresponding angle information; and
a selection module configured to show the searched out organic chemical structures in the input window and provide codes for selection, when a plurality of organic chemical structures are searched out.

Preferably, the apparatus may further comprise:
an interpretation module configured to interpret the character string as a operating command when the focus are a plurality of atoms and/or bonds; and
an execution module configured to execute the operating command for the plurality of atoms and/or bonds

From the above, according to the above embodiments of the present invention, efficiency of typesetting can be improved and learning difficulty can be reduced.

It will be readily apparent to those skilled in the art that respective modules or steps of the present application may be implemented with a common computing device. In addition, the modules or steps of the present application may be concentrated in a single computing device or distributed in a network composed of multiple computing devices. Optionally, the modules or steps may be achieved by using codes of executable programs, so that they can be stored in a storage medium, or can be respectively fabricated into various individual integrated circuit modules, or the plurality of the modules or steps can be fabricated into one individual integrated circuit module. Therefore, the present application is not limited to any particular hardware, software or combination thereo f.

The foregoing is only preferred embodiments of the present application, and it is not intended to limit the present application. Moreover, it will be apparent to those skilled in the art that various modifications and variations can be made to the present application. Thus, any modifications, equivalent substitutions, improvements etc. within the spirit and principle of the present application should be included within the scope of protection of the application.

## Claims

1. A method for constructing an organic chemical structure formula comprising:
acquiring, through a keyboard, a character string inputted by a user;
searching, in a preset code mapping set, an organic chemical structure mapped by the acquired character string; and
constructing the organic chemical structure formula according to the searched organic chemical structure.

2. The method according to claim 1, wherein the step of acquiring comprises:
providing an interactive chemical typesetting interface;
activating an input window in the interface; and
monitoring inputs from the keyboard.

3. The method according to claim 2, further comprising:
presetting the code mapping set including a plurality of metadata, wherein the metadata comprises:
a structure type item for indicating that a focus is no-focus, an atom or a bond;
a code mapping item for indicating a character string mapping the organic chemical structure; and
a structure data item for indicating the mapped organic chemical structure.

4. The method according to claim 3, wherein the step of searching comprises:
determining that the current focus in the interactive chemical typesetting interface is no-focus, an atom or a bond;
looking up the structure type item and the code mapping item in the code mapping set according to the determined focus type and the acquired character string; and
extracting the organic chemical structure from the structure data item of the metadata.

5. The method according to claim 4, wherein in the step of constructing,
when it is determined that the focus is no-focus, an organic chemical structure in the structure data item from the interactive chemical typesetting interface is outputted;
when it is determined that the focus is an atom, the step of constructing is implemented by one of the following connection modes:
a many-to-many connection mode in which a new chemical bond is constructed between the atom at the focus and an atom of the searched organic chemical structure, the bond length of the new chemical bond is determined as a default bond length, and then the angle position of the new chemical bond is determined according to the position of the atoms in the interactive chemical typesetting interface and information of the connected bonds;
an one-to-one connection mode in which the atom at the focus and the chemical bond connected thereto are deleted and replaced with contents of the organic chemical structure; and
a many-to-one or one-to-many connection mode in which the atom at the focus and an atom of the searched organic chemical structure are connected and combined directly, and the position and angle of the connected atoms are determined according to information in the interactive chemical typesetting interface; or
when it is determined that the focus is a bond, the bond and the searched organic chemical structure are connected to be a new bond, the bond length of the new bond is determined according to information of the bond at the focus and then the position of the new bond is determined according to the corresponding angle information; and
wherein the searched organic chemical structures are shown in the input window and codes are provided therein for selection, when a plurality of organic chemical structures are searched out.

6. The method according to claim 1, further comprising:
interpreting the character string as a operating command when the focus are a plurality of atoms and/or bonds; and
executing the operating command for the plurality of atoms and/or bonds.

7. An apparatus for constructing an organic chemical structure formal comprising:
an acquiring module configured to acquire a character string inputted through a keyboard by a user;
a searching module configured to search, in a preset code mapping set, an organic chemical structure mapped by the acquired character string; and
a constructing module configured to construct the organic chemical structure formula according to the searched organic chemical structure.

8. The apparatus according to claim 7, wherein the acquiring module comprises:
an interface module configured to provide an interactive chemical typesetting interface;
a window module configured to activate an input window for the keyboard;
and
a monitor module configured to monitor inputs from the keyboard.

9. The apparatus according to claim 8, further comprising:
an encoding module configured to preset the code mapping set including a plurality of metadata, wherein the metadata includes
a structure type item for indicating a focus being no-focus, an atom or a bond;
a code mapping item for indicating a character string mapping an organic chemical structure; and
a structure data item for indicating an mapped organic chemical structure.

10. The apparatus according to claim 9, wherein the searching module comprises:
a focus module configured to determine whether the current focus in the interactive chemical typesetting interface is no-focus, an atom or a bond;
a lookup module configured to search the structure type item and the code mapping item in the code mapping set according to the determined focus and the acquired character string; and
an extracting module configured to extract the organic chemical structure from the searched structure type item of the metadata.
